# EUROPEAN PATENT SPECIFICATION

(11) **EP 0 242 225 B1**
(45) Date of publication and mention of the grant of the patent: **18.11.1993**
(21) Application number: 87303411.0
(22) Date of filing: 16.04.1987
(51) Int. Cl.: G01N 33/18

(54) **Pollutant detector**
Schadstoffdetektor
Détecteur d'agents polluants

(30) Priority: 16.04.1986 GB 8609273; 11.02.1987 GB 8703119
(43) Date of publication of application: 21.10.1987
(73) Proprietor: WATER RESEARCH CENTRE, Marlow Buckinghamshire SL7 2HD (GB)
(72) Inventor: Rawson, David Michael, Luton Bedfordshire LU2 7HW (GB)
(74) Representative: Wain, Christopher Paul

(56) References cited:
- GB-A- 2 000 805
- US-A- 3 403 081
- US-A- 3 479 255
- US-A- 3 506 544

## Description

The present invention relates to a pollutant detector and a method of detecting pollution. More particularly, but not exclusively, the invention relates to a method and apparatus for detecting pollution in aqueous liquids, for example water supply, by use of a bacterial sensing means.

Thirty percent of water supplies in the United Kingdom are drawn directly from rivers, which may on occasion be subject to pollution. Such pollution may cause shut down of the water purification works if detected in time. If the pollution is not detected in time, the water supply may be contaminated. Many of the pollutants which may enter rivers are toxic chemicals, e.g. herbicides, which may not degrade during the normal water purification process.

Typical pollutants which may be found in water supplies and which may be detected by the present invention include:

| | |
|---|---|
| Diesel oil | Oil |
| Petroleum | Aniline |
| Metal Salts e.g. of Cd,Cu,Cr | Agricultural chemicals |

and particularly such commercial herbicidal formulations as:

| | |
|---|---|
| DIQUAT | PARAQUAT |
| MORPHAMQUAT | BROMOXYNIL |
| DICHLOBENIL | DIPHENATRILE |
| IOXYNIL | AMETRYNE |
| ATRATONE | ATRAZINE |
| AZIPOTRYN | BLADEX |
| CHLORAZINE | CYPRAZINE |
| DESMETRYNE | IPAZINE |
| METHOPROTYN | PROMETRONE |
| PROMETRYNE | PROPAZINE |
| SIMAZINE | SIMETONE |
| SIMETRYNE | TERBUTRYN |
| TRIETAZINE | BENZOMARC |
| BENZTHIAZURON | BUTURON |
| CHLOROBROMURON | CHLOROXURON |
| CHLORTOLURON | CYCLURON |
| DCU | DIFENOXURON |
| DIURON (DCMU) | FENURON |
| FLOUMETURON | ISONORURON |
| KARBUTILATE | LINURON |
| METHABENZTHIAZURON | METOBROMURON |
| METOXURON | MONOLINURON |
| MONURON | MONURON-TCA |
| NEBURON | NOREA |
| SIDURON | TRIMETURON |
| ACROLEIN | PYRAZON |
| 6706 | 9785 |
| 9774 | BROMOCIL |
| LENACIL | DP-733 |
| ISOCIL | TERBACIL |

There is a need for rapid, simple and low cost toxicity screening procedures which can be used to assess the impact of the increasing number of chemicals on the quality of aquatic and soil environments. This need is seen nowhere more acutely than at intake protection for drinking water, especially when raw water is being abstracted from lowland rivers at risk from industrial or agricultural pollution. Such water may, in some circumstances, be collected, treated and piped to a householder in less than four hours. In such situations monitors must be capable of responding rapidly, perhaps within 30 minutes.

The success of the Ames test for rapid screening of chemical mutagens has stimulated research on the use of bacteria for rapid and inexpensive screening of chemical

During recent years, the use of microorganisms in aquatic tests has received increasing attention. Techniques are becoming more diversified and have been developed to fulfill various screening functions. Amongst these are the Spirillum volutans inhibition test, the triphenyltetrazoliumchloride (TTC), and resazurin, dehydrogenase activity inhibition tests, the Microtox assay protocol and the activated sludge respiration inhibition test. Although enjoying some degree of success, these tests suffer from two major disadvantages. First they require a certain amount of skilled manipulation and are therefore time consuming. Secondly, they cannot easily be applied on line thus making real time analysis impractical. Biosensors, which are devices that transduce a selective biochemical response into an electrical signal can offer practical alternatives in environmental monitoring particularly in terms of cost, ease of manufacture, ability to reduce the test system's complexity to a minimum and suitability for on line applications. A further advantage offered by biosensors is that a specific determination can be carried out in multi-component solutions thereby alleviating the need for any complicated and time consuming separation procedures.

In general terms a biosensor consists of a biological component such as an enzyme, tissue slice or whole cell held in close proximity to the surface of a transducing element. In this configuration the biocatalyst not only confers selectivity on the device but also produces or consumes a species which can be detected by the transducer.

Carbon dioxide specific electrodes have been used in conjunction with immobilised bacteria to produce a whole cell biosensor capable or toxin detection. This particular approach makes the possibility of on-line monitoring more feasible. Where herbicide disturbance of photosynthetic electron transport (PET) systems is being monitored, the biocatalyst must incorporate complete photosystems capable of carrying out the Hill reaction. Isolated thylakoids, chloroplasts and intact photosynthetic prokaryotic or eukaryotic cells are capable of acting as such biocatalysts. However, complexities of preparation and poor stability of isolated membranes and organelles reduce the attractiveness as potential biocatalysts. Cyanobacterial and algal cells, however, are easily maintained in axenic culture and harvested to give uniform batches of material. The absence of membrane bound organelles in cyanobacteria makes these organisms particularly suitable for biosensor use.

Various proposals have been made in the past for testing fluids using bacteria or algae. For example, US-A-3403081 describes a biosensor with a reference electrode and a bioelectrode including a micro-organism or enzyme, the presence of contaminant being evidenced by a change in the electrical potential of the bioelectrode relative to the reference electrode. US-A-3506544 describes the use of enzyme-catalysed reactions in the presence of a redox couple to give an electrical indication of the enzyme reaction using methylene blue as an electron transfer mediator. US-A-3479255 describes a system including living bacteria (or algae) whose activity is reduced by the presence of poisons. The activity change is sensed using an ion-sensitive surface whose electrical characteristics are changed in dependence on the bacterial activity. GB-A-2000805 describes a method of studying cell behaviour in which essential nutrients are provided and an electron transporter to intervene in a metabolic route of the cell activity.

The use of whole cells in biosensors offers the advantages of increased stability and ease of immobilisation. Mediators, such as soluble, low molecular weight redox couples, are provided to interact at sites along the ET chain and become reduced, in effect acting like terminal electron acceptors. Subsequent reoxidation at the working electrode results in a steady flow of current which is measured by the external circuitry. The magnitude of the current is proportional to the photosynthetic activity of the organisms and any perturbations in the ensuing current/time curve are used to indicate the presence of a pollutant.

We have now found that certain electron transfer mediators are particularly useful in a bacteria/algae biosensor.

According to the present invention, there is provided a method of detecting pollution in an aqueous liquid flow comprising the steps of: adding to a portion of the liquid a compatible electron transfer mediator, passing the mixture into a sensor cell in which is contained living bacteria or algae, stimulating an activity of said bacteria or algae, and measuring the level of said activity at an electrode in the cell by means of electron transfer thereto by said mediator; characterised in that said mediator is potassium ferricyanide, dimethylbenzoquinone or p-benzoquinone either alone or in admixture, and in that the activity monitored of the bacteria or algae is respiration and/or photosynthesis.

The invention also includes a sensor to detect the presence of a pollutant in an aqueous liquid, which sensor comprises an electrode (4), living bacteria or algae, means to stimulate activity of the bacteria or algae (6), a compatible mediator to transmit an indication of the activity level of the bacteria or algae to said electrode, and means to measure an electrical parameter of said electrode for determination as to normality of the bacteria/algae activity; characterised in that said mediator is potassium ferricyanide, dimethylbenzoquinone or p-benzoquinone either alone or in admixture.

In accordance with the invention, the activity monitored of the bacteria or algae is respiration and/or photosynthesis, both of which involve electron transfer.

The mediator preferably operates by electron transfer from the respiratory or photosynthetic pathway of the bacteria or algae to the electrode, the current flowing from which acts as a measure of the bacteria activity.

The mediator may be potassium ferricyanide, dimethylbenzoquinone or p-benzoquinone, either alone or in admixture.

The preferred bacteria are cyanobacteria, for example Synechococcus, or alternatively Eubacteria, for example, E.coli.

An alternative is a eukaryotic alga, for example chlorella.

The means to stimulate activity of the bacteria or algae may be a light source, for example a bright light emitting diode disposed close to the bacteria or a high intensity light source and light guide.

The bacteria or algae may be retained on a translucent alumina filter. In this case, the bacteria may be held by means of a fine nylon mesh.

Alternatively, the bacteria or algae may be provided with an energy source, for example glucose and/or fructose.

A determination of normality may be made by comparison with the electrical parameter output of a control electrode in a sensor having aqueous liquid of predetermined purity.

The electrical current may be measured as derived preferably by means of a carbon or platinum electrode poised in the region of 400-550 mV with reference to silver/silver chloride.

The bacteria or algae may be in a 0.2 µm bacteriological filter membrane which may be held against electrode surface by nylon mesh. Alternatively, they may be held in a reservoir through which the aqueous liquid and mediator are passed prior to their passing to the electrode.

Embodiments of the invention will now be more particularly described by way of example and with reference to the accompanying drawings, in which:
FIGURE 1 shows diagrammatically a form of flow cell for use in the invention;
FIGURE 2 shows schematically the electron transfer events during photosynthesis of cyanobacteria and the sites of access of mediators;
FIGURE 3 shows graphically the results of a series of control sensor runs with various combinations of bacteria/alga and mediator with LED stimulation;
FIGURES 4 and 5 show continuations of the runs of Figure 3 with the addition of a pollutant;
FIGURE 6 shows a graphical comparison of a control run and a run with the addition of DCMU pollutant for a Synechococcus sensor;
FIGURE 7 shows graphically the results of a control sensor run of E.coli and mediator with glucose stimulation;
FIGURE 8 shows an electrochemical cell which may be magnetically stirred;
FIGURE 9 shows schematically a tangential flow electrochemical cell for online use;
FIGURE 10 shows graphically the results of photosynthetic activity monitored in Synechococcus (a) and Chlorella (b) immobilised on cellulose acetate filters and mounted in the flow cells, using ferricyanide and ferricyanide/p-benzoquinone mediators. The working potentials in both instances are 400 mV vs the Ag/AgC1 reference electrode. Illumination was provided by LEDs; (+) = LEDs on, (-) = LEDS off;
FIGURE 11 shows graphically the response of a Synechococal biosensor incorporating cells harvested from cultures in stationary phase, immobilised onto alumina filters, and incorporated in a flow cell with a mediator solution of ferricyanide in Bgll. Again illumination was provided by LEDs; (+) = LEDs on, (-) = LEDs off. The working electrode potential was 400 mV vs Ag/AgCl. (The spike is the result of introducing an air bubble into the mediator flow stream to the sensor.);
FIGURE 12 shows graphically the effect of the herbicide DCMU on the response from a Synechococcal biosensor using cells immobilised on cellulose acetate filters with ferricyanide as the mediator. DCMU was added to the reservoir (+DCMU) to give a final herbicide concentration of 233 ppb. Both sensors experienced the same light and dark regimes, illumination being by LEDs; (+) = LEDs on. (-) = LEDs off. The potential at the working electrode was 400 mV vs Ag/AgCl;
FIGURE 13 shows graphically the effect of chlortoluron on the response from a Synechococcal electrode, using cells immobilised on alumina filters with potassium ferricyanide as the mediator. (+) = illumination on, (-) = illumination off and (+Chl) = an addition of chlortoluron to the electrochemical cell resulting in a final concentration of 2 ppm;

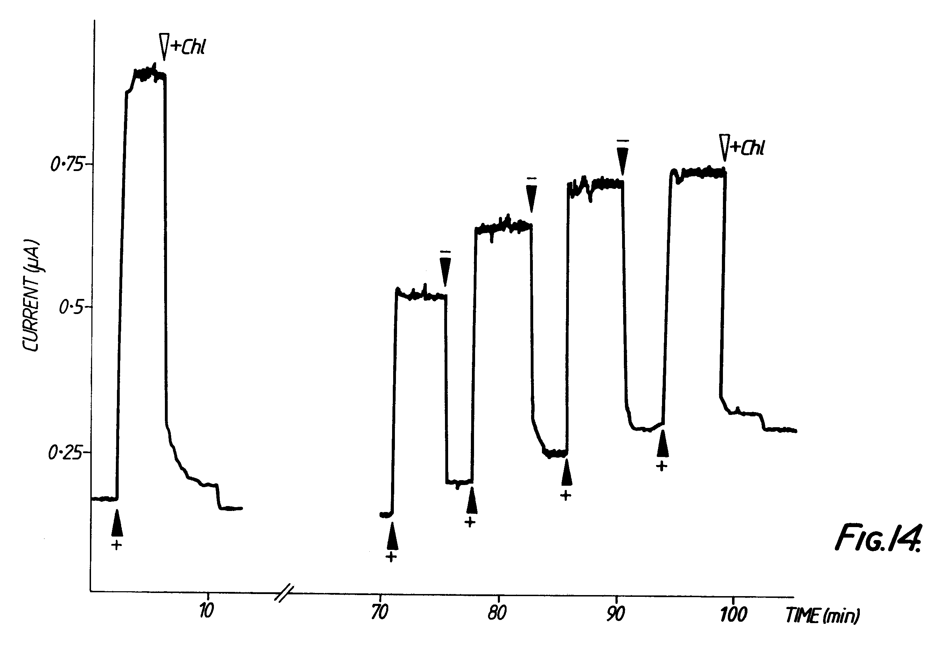

FIGURE 14 illustrates graphically reversible inhibition by the herbicide chlortoluron of a Synechococal biosensor. The cells are immobilised onto alumina filters and the sensor was operated in the stirred glass cell using ferricyanide as mediator at a working potential of 550mV vs Ag/AgCl. Following a one hour flushing of the sensor in mediator free Bgll repression by chlortoluron was partially reversed. Photosynthetic electron transport is again monitored using ferricyanide and a second response to chlortoluron obtained. Chlortoluron was added at (+Chl) to give a final concentration of 2ppm; and
FIGURE 15 shows graphically the effect of linuron on the response obtained from a whole cell biosensor incorporating Synechococal cells immobilised onto alumina filters, with ferricyanide as mediator and poised at 550 mV vs a silver chloride reference. Illumination was provided by an external light source; (+) = light on, (-) = light off. Linuron additions (+ Lin) were made as indicated resulting in final concentrations of 17 ppb and 34 ppb.

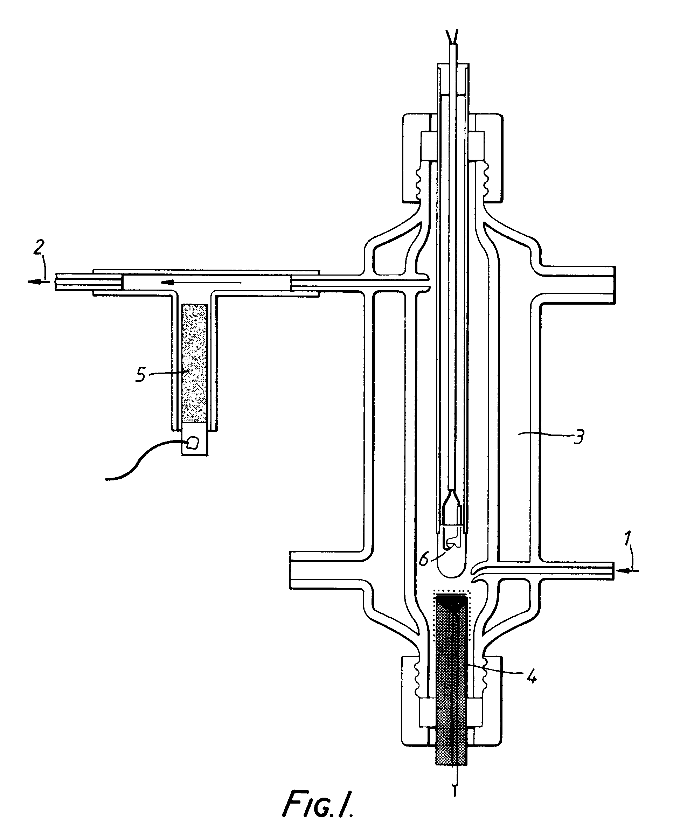

Referring now to Figure 1, a sensor cell comprises an inlet 1 and outlet 2 for passage of aqueous liquid mixed with mediator. A water jacket 3 is provided to maintain an even temperature, if this should be desired. The liquid flows over an electrode 4 which is a carbon or platinum electrode poised at 400 mV with respect to a silver/silver chloride reference electrode 5.

An intense LED 6 is disposed adjacent the electrode 4, spaced in a preferred embodiment 3mm therefrom. Fine nylon mesh holds a 0.2 µm bacteriological filter against the electrode 4. Captured on the filter, on the illuminated side, i.e. remote from the electrode, and protected by a dialysis membrane, is a bacterium or alga which may be as follows (PCC means Pasteur Culture Collection):

| | |
|---|---|
| Synechococcus | PCC - 6301 |
| Anabaena cylindrica | PCC - 7122 |
| Anabaena Variabilis | ATCC - 29413 |
| Chlorella sp. | |
| Escherichia coli | |
| Bacillus licheniformis | |
| Bacillus subtilis | |
| Agrobacterium tumefaciens | |

The first four of the above bacteria may be stimulated by illumination of the LED, or the final six of the bacteria may be stimulated by adding glucose and/or fructose to the input liquid.

The sensor cell is arranged with a number of others to receive selectively a portion of the input liquid. The effective life span of the bacteria varies with species, age and metabolic status, but will require renewal after a number of hours or days, use. In order to provide continuous detection, the input flow can be switched to a renewed cell while the bacteria means in a "used" cell is changed. Further cells can be provided to act as control cells to which liquid of known or standard purity is fed. The cells may be renewed by exchanging a fresh supply of bacteria for the used bacteria or alternatively by supplying the cell with a saline or mineral solution containing nutrient over a "recovering phase", the length of which may vary.

The mediator mixed with the input is potassium ferricyanide, dimethylbenzoquinone and/or p-benzoquinone.

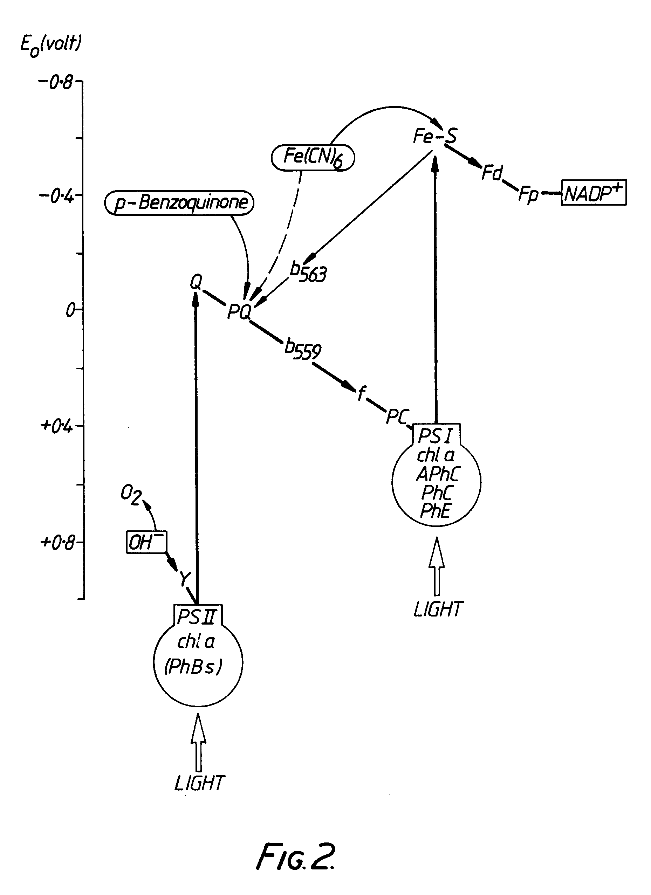

Figure 2 shows schematically the electron transfer events during photosynthesis by cyanobacteria. The electrons captured by the mediator are transmitted to the electrode 4 where the current can be measured.

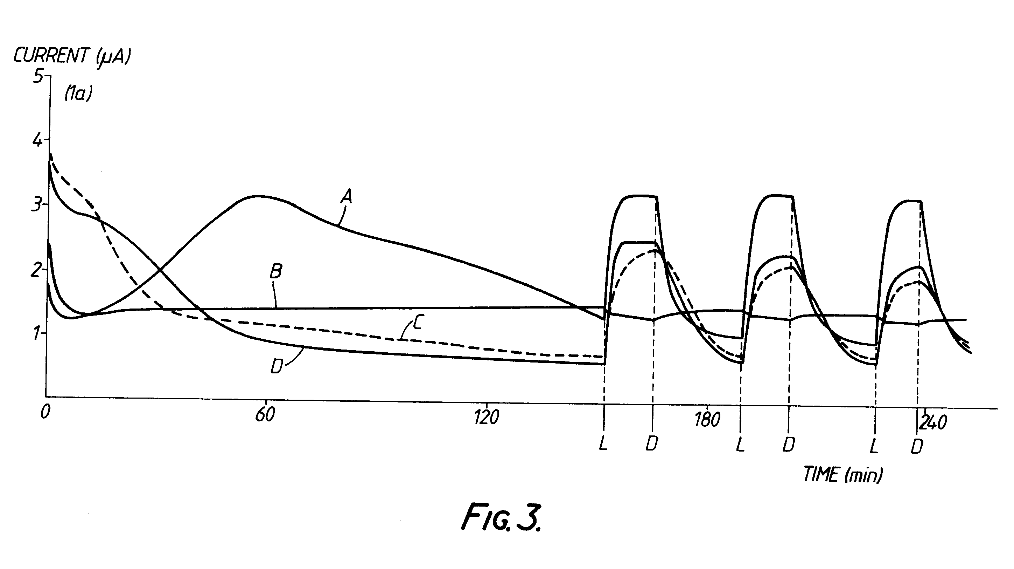

Referring now to Figures 3 to 5, four runs are plotted graphically as follows:
- A -: Cyanobacterium - Synechococcus with 5mM potassium ferricyanide mediator;
- B -: Eukaryotic alga-chlorella also with 5mM potassium ferricyanide mediator;
- C -: Synechococcus with mixture of 5mM ferricyanide and 0.5 mM p-benzoquinone; and
- D -: Chlorella with the same mediator mixture as in C.

At intervals, once the bacteria or algae had a stabilised activity, the LED was illuminated, as indicated by points L. It was switched off again at D. As can be seen, in all except one case, illumination produced increased activity (as measured at the electrode) fairly quickly.

At dimming the activity, again with the one exception, diminishes. The pattern in each case is however, regular. The exceptional case would seem to show that eukaryotic alga will not function properly in the absence of p-benzoquinone.

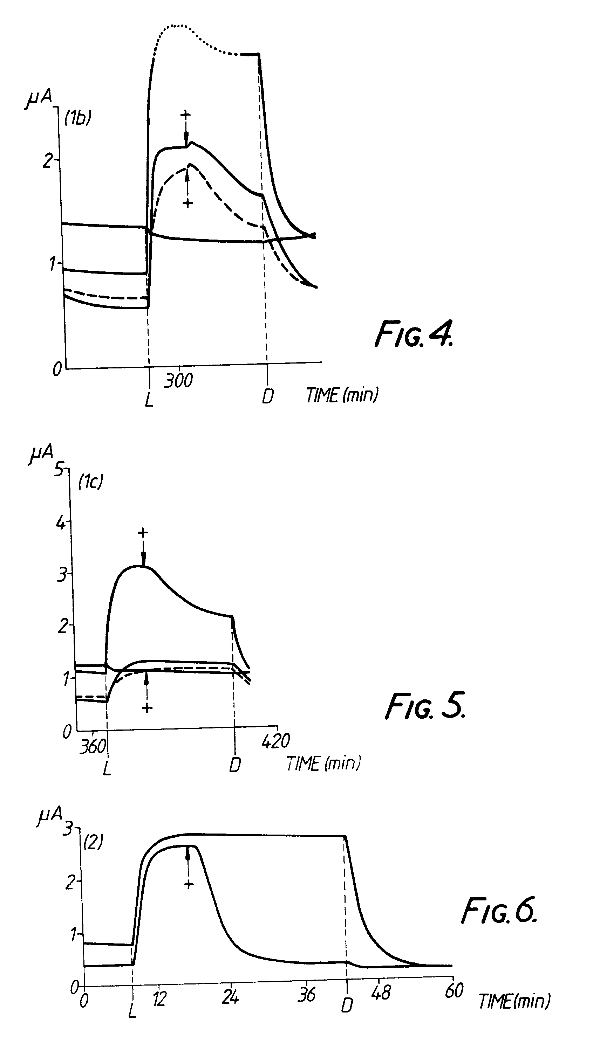

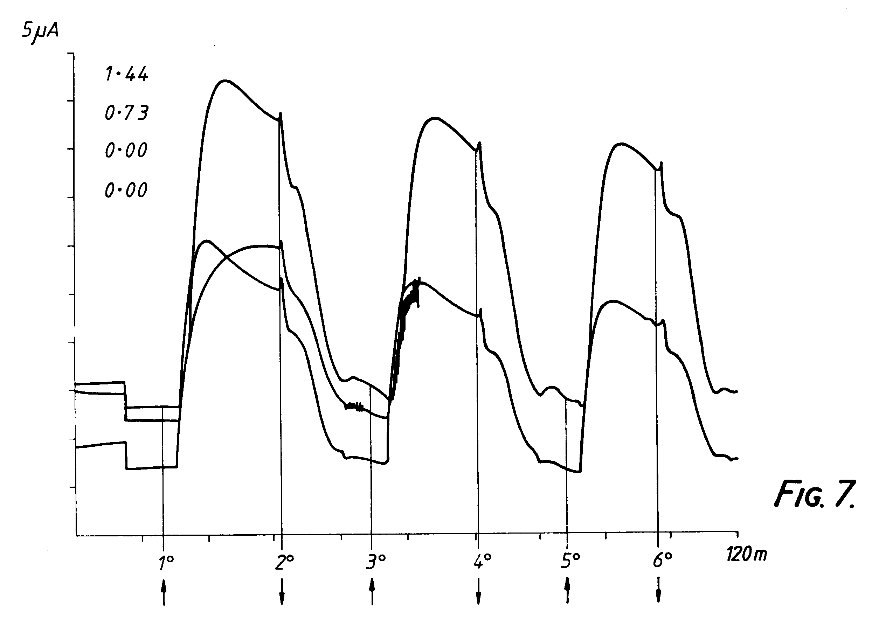

In Figures 4 and 5, a herbicide 3-(3,4-dichlorophenyl)-1,1-dimethylurea (DCMU) was added to a mediator reservoir at the points marked +, to give a final concentration of 0.75 x 10⁻⁶ M (c. 175 ppb). The effects can be seen clearly from the Figures.

The effect is shown even more clearly in Figure 6 which shows the effect of addition of 1 x 10⁻⁶ M (c. 233 ppb) DCMU at the point marked + to a Synechococcus sensor, as opposed to a control Synechococcus sensor. The addition gives a marked reduction of bacterial activity within 2 or 3 minutes.

Finally Figure 7 shows graphically the activity of a Eubacteria - Escherichia coli as transferred by a ferricyanide mediator, when activated at intervals at points ↑ by addition of glucose to the input, and deactivated at ↓ by returning the input to a mediator/saline mixture.

The method and apparatus disclosed provide a useful means of determining quickly whether or not a pollutant is present in the water intake. When a signal is received that a pollutant may be present, the flow can be interrupted and the nature of the pollutant determined by analytical or other techniques.

The invention has been described with reference to particular bacteria and algae. Others may of course be applicable, as may other compatible mediators.

Since the mediator transfers the activity of the bacteria to the electrode, it is not strictly necessary for the bacteria to be immediately adjacent the electrode. The bacteria may be contained in a reservoir remote from the electrode and the mediator/input liquid passed from the reservoir to the electrode.

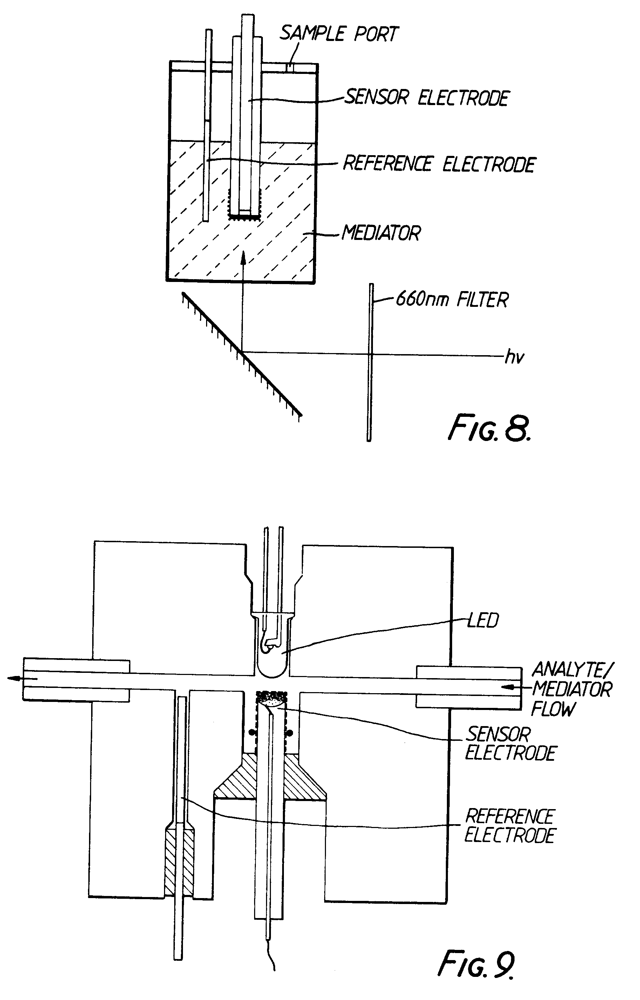

Figure 9 shows a tangential flow electrochemical cell in which the analyte, mixed with a mediator, flows across a sensor electrode while being periodically illuminated by means of the LED.

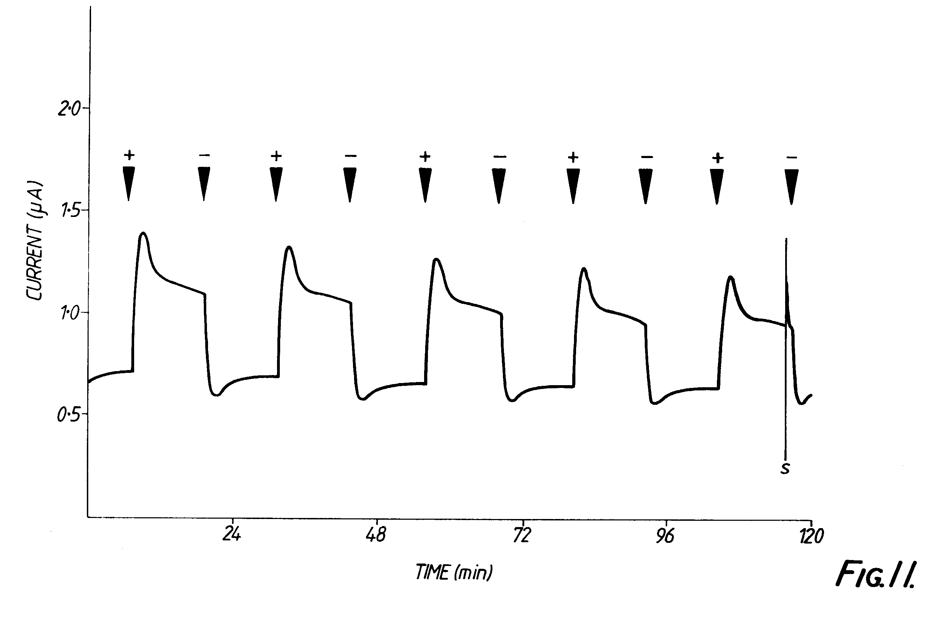

The remaining Figures show graphically the results of using various bacteria or algae in the method of the invention. One point to note is that, as shown in Figure 11, an air bubble may cause a spike. Thus it is advisable that the sample should first pass through a bubble trap to eliminate any such complications.

It is possible to maintain the shelf life of the bacteria or algae used for a period of up to many months. The cells can be presented to the sensor electrode captured in calcium alginate. Bacterial or cyanobacterial cells may be harvested during logarithmic growth and mixed with sodium alginate in a concentration in the range of 1-4% weight/vol. to give the desired cell density. The sodium alginate is then solidified, by imersion in 0.5 molar calcium chloride solution at 4°C. for six hours, into beads or sheets. Following rinsing in distilled water, the alginate is air dried for twelve hours at 25°C. and stored in sealed glass containers until needed.

Prior to use on the sensor electrodes, the alginate is re-hydrated in an appropriate bathing medium (such as BG 11, or 0.9% saline solution).

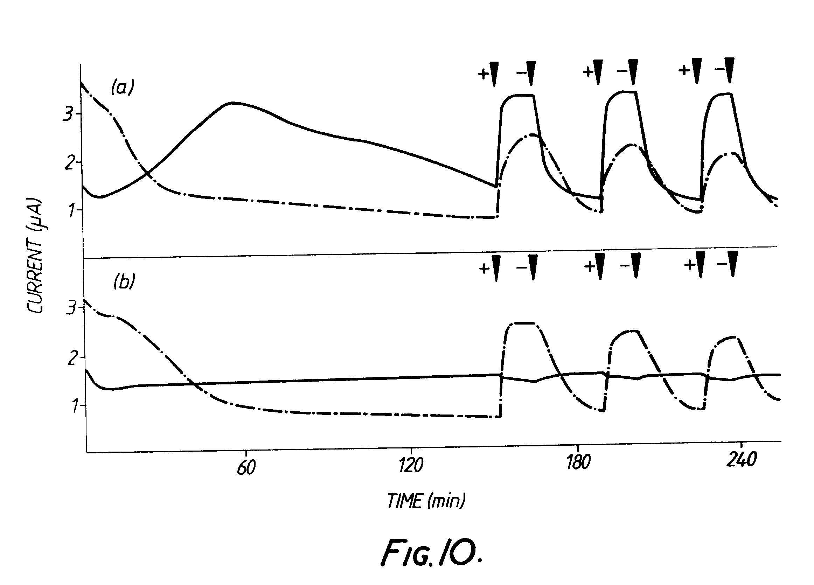

Referring now more particularly to Figure 10, the response obtained with Synechococcus and Chlorella based biosensors to a regime of light and dark periods in the presence of either ferricyanide or a mixture of ferricyanide and p-benzoquinone is shown. The inability of ferricyanide to access eukaryotic photosynthetic activity is clearly seen as no response is recorded from the Chlorella electrodes following illumination. A cocktail containing a lipophilic mediator is required to access the chloroplast ET chains. It is believed that benzoquinone alone will act as an efficient mediator in this system.

With both Synechococcus and Chlorella based sensors, the use of p-benzoquinone results in a sequential decline in the maximum current obtained during the period of illumination, ultimately resulting in total loss of response.

In the case of the Synechococcus biosensors, PET chain events can be monitored using ferricyanide alone, with a less marked decline in the light response. A working life for Synechococcal biosensors of 3-4 days continuous use, if not more, can be achieved using ferricyanide.

Typically with Synechococcus, if cells are used immediately after harvesting from illuminated batch culture, a substantial dark respiratory response is observed (early portion of the curve) which must be exhausted before the sensor is used for photosynthetic monitoring. This response is not seen however, with Chlorella or indeed in Synechococcus when p-benzoquinone is present in the electrolyte.

Of the three cyanobacteria tested in this investigation, Synechococcus was found to be the most successful, whilst PET activity could be monitored in both Anabaena cylindrica and Anabaena variabilis, the use of these filamentous forms proved more difficult in terms of obtaining optimum loading levels on the filters. In addition, the response times were longer with these organisms than with Synechococcus. Based on these and other results the unicellular cyanobacterium Synechococcus PCC 6301 was selected as the biocatalyst for use in all subsequent work.

Figure 11 shows typical results obtained from a Synechococcus probe produced from cells harvested from cultures during their stationary phase of growth. Following illumination, such sensors showed a rapid increase in current which reached a maximum peak value. The current then declined to a lower but more stable value. This type of overshoot effect, which is only seen with cells harvested from older cultures, is evident in all subsequent light stimulations. The magnitude of the overshoot together with the steady portion of the curve is consistent throughout the sensor run. The spike towards the end of the sensor run demonstrates the effect of an air bubble introduced into the flow stream. In order to guard against this during on line applications, a bubble trap on the delivery line to the flow cell needs to be fitted.

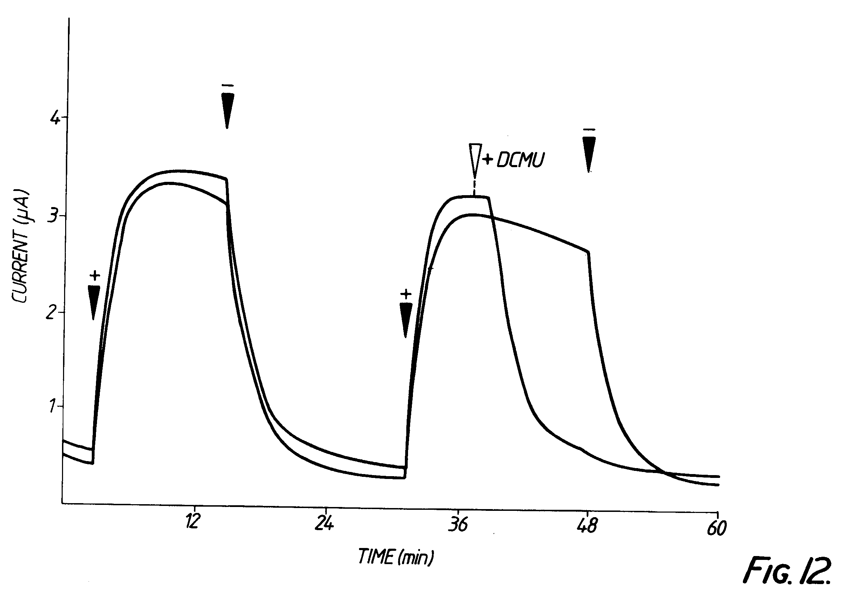

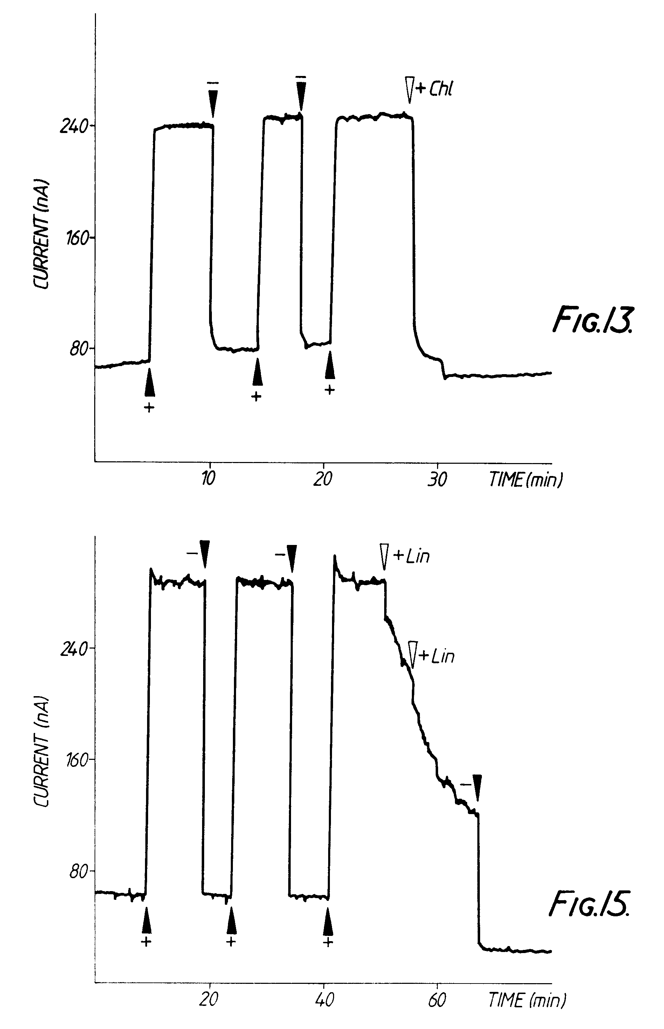

To confirm that the sensor response was indeed the result of PET, the effects of DCMU, a known cyanobacterial photosynthetic inhibitor was investigated. Using Synechococcus biosensors in the tangential flow cell configuration, the response to the addition of 1x10⁻⁶ M (233 ppb) DCMU was compared to a control sensor, as is shown in Figure 12. Following a short transit time taken to deliver the herbicide to the biosensor from the receiving reservoir, there was a rapid cessation in PET activity, as evidenced by the drop in sensor current which mirrors that seen on the removal of illumination to the control sensor. So long as the DCMU is present in the flow stream no recovery in the light response could be obtained from the "poisoned sensor". Subsequently, two other members of the DCMU family of herbicides, chlortoluron and linuron, were investigated. The effect of an addition of chlortoluron to give a final herbicide concentration of 2 ppm, on a Synechococcus biosensor shows in Figure 13 an equally rapid fall in photosynthetic ET activity.

The reversibility of the effects of these herbicides is shown in Figure 14 where chlortoluron inhibition of Synechococcal photosynthesis at a concentration of 2 ppm is reversed following a one hour recovery period during which the sensor is flushed with fresh Bgll medium. Similar results, both in terms of inhibiting the photosynthetic response and recovery after washing were obtained for the herbicides DCMU and linuron.

Figure 15 shows the results of a two pulse addition of Linuron, final concentration 17ppb and 34ppb. Although the decrease in current is not as rapid as that seen for higher concentrations (200 ppb - 2 ppm) it is nevertheless substantial and can be used as an indication of the presence of herbicide pollution, even at these extremely low levels.

In order to ensure the successful detection of a particular herbicide, it is important to choose the correct mediator molecule. In order to detect the presence of a PSII inhibitor for example, the mediator must be reduced downstream from the herbicide's site of action. Similarly, in order to monitor the presence of a PSI blocker, the mediator should be reduced at the distal end of the PET i.e., near the terminal electron acceptor site. In this context ferricyanide is a good choice for a mediator, not only because of its chemical stability in aqueous solution and straightforward electrochemistry at the working electrode, but also because it interacts with the PET at the end of PSI. Consequently, by using ferricyanide as the mediator it should be possible to monitor all herbicides which prevent the flow of electrons through PSII and those which interrupt electron flow through PSI before the ferrodoxin, the putative ferricyanide reduction site. These sensors may be applied to detecting a wide range of herbicide families such as nitriles, triazines, bis-carbamates, pyridazines, nitrophenyl ethers, uracils and anilides.

The sensor configuration shown in Figure 9 could have an important role in detecting pollution plumes in river water, and is intended to supplement existing monitoring systems. Biosensors have the advantages of being cheap to produce, the sensor electrode being a disposable device, and requiring minimal pretreatment, i.e. removal of suspended solids and air bubbles from the sample flow to the sensor. The system can also be fully automated, opening up the possibility of locating sensors at remote sites.

The invention will be further described with reference to the following Example.

### EXAMPLE

Carbon foil indicator electrodes were manufactured in the following manner. 5mm diameter discs were punched out of a 1mm thick strip of porous graphite, washed twice in acetone (30 minutes for each wash with gentle agitation), once in boiling distilled water (1 hour), and dried in air oven at 100°C. Electrical contact to the discs was made by cementing lengths of 0.2mm wire to the surface of the graphite with a drop of electrically conducting (silver loaded) epoxy resin. After curing, the graphite discs were potted in a mixture of 9 parts epon resin (grade 815) and 1 part triethylenetetramine catalyst at a temperature of 60°C. Immediately prior to use, the electrodes were conditioned electrochemically in 0.1 M potassium dihydrogen orthophosphate by cycling the potential between -0.8 and +1.0V vs Ag/AgCl 100 times at a sweep rate of 1V/sec.

Anabaena variabilis (ATCC 29413) and Synechococcus (PCC 6301), Anabaena cylindrica (PCC 7122) and Chorella sp. were used. Cultures of anabaena were maintained on agar plates made from BG11o medium, nitrate-free BG11 medium supplemented with 1.5% (w/v) bacteriological agar. Synechococcus and Chlorella cultures were maintained on agar supplemented BG11 medium. Liquid batch cultures were prepared by transfer of inoculum from plate cultures to 50 ml sterile BG11o or BG11 medium in conical flasks. Liquid cultures were maintained at 25°C., with 5.0 W m⁻² illumination to a mid exponential phase (OD₆₆₃= 1.0) and the cells were harvested by rapid centrifugation (12,000 rpm for 60 secs.). The cells were resuspended in 50µl of fresh culture medium and applied to the surface of 5mm diameter bacteriological filter discs.

Two different techniques were employed in the present Example. Initially cells were loaded onto 0.2µm cellulose acetate filter discs. The filters were then placed onto the sensor electrode with the cell loaded surface exposed. Dialysis membrane was used to sheath the electrode and hold the filter and cells in place. Alumina filters which are translucent when wet can now be used to replace cellulose acetate. In this case the alumina filters arepresented to the electrode with the cell layer against the electrode surface. The filter is held in place by a fine nylon mesh, removing the need for a dialysis membrane. Prior to use, the assembled electrodes were conditioned by soaking in BG11 medium for approximately 10 minutes.

## Claims

1. A method of detecting pollution in an aqueous liquid flow comprising the steps of: adding to a portion of the liquid a compatible electron transfer mediator, passing the mixture into a sensor cell in which is contained living bacteria or algae, stimulating an activity of said bacteria or algae, and measuring the level of said activity at an electrode in the cell by means of electron transfer thereto by said mediator; characterised in that said mediator is potassium ferricyanide, dimethylbenzoquinone or p-benzoquinone either alone or in admixture, and in that the activity monitored of the bacteria or algae is respiration and/or photosynthesis.

2. A method as claimed in claim 1, characterised in that the mediator operates by electron transfer from the respiratory or photosynthetic pathway of the bacteria to the electrode, the current flowing from which acts as a measure of the bacteria activity.

3. A method as claimed in claim 1 or 2, wherein the bacteria are cyanobacteria, preferably Synechococcus.

4. A method as claimed in claim 1 or 2, wherein the bacteria are Eubacteria, preferably E.coli.

5. A method as claimed in claim 1 or 2, wherein the algae are eukaryotic algae, preferably chlorella, and the mediator is not potassium ferricyanide alone.

6. A method as claimed in claim 3 or 5, wherein the means to stimulate activity of the bacteria or algae, is a light source, preferably a bright light emitting diode disposed close to the bacteria or algae.

7. A method as claimed in claim 4 or 5, wherein the bacteria or algae activity is stimulated by provision of an energy source, preferably addition of glucose and/or fructose.

8. A method as claimed in any preceding claim, wherein the bacteria or algae are retained on a translucent alumina filter.

9. A method as claimed in any preceding claim, wherein a determination of normality is made by comparison of an electrical parameter output of the electrode with that of a control electrode in a sensor having aqueous liquid of predetermined purity.

10. A sensor to detect the presence of a pollutant in an aqueous liquid, which sensor comprises an electrode (4), living bacteria or algae, means to stimulate activity of the bacteria or algae (6), a compatible mediator to transmit an indication of the activity level of the bacteria or algae to said electrode, and means to measure an electrical parameter of said electrode for determination as to normality of the bacteria/algae activity; characterised in that said mediator is potassium ferricyanide, dimethylbenzoquinone or p-benzoquinone either alone or in admixture.

11. A sensor as claimed in claim 10, wherein the bacteria are cyanobacteria, preferably Synechococcus.

12. A sensor as claimed in claim 10, wherein the bacteria are Eubacteria, preferably E.coli.

13. A sensor as claimed in claim 10, wherein the algae are eukaryotic algae, preferably chlorella, and that the mediator is not potassium ferricyanide alone.

14. A sensor as claimed in claim 11 or 13, wherein the means to stimulate activity of the bacteria or algae is a light source, preferably a bright light emitting diode (6) disposed close to the bacteria or algae.

15. A sensor as claimed in claim 12 or 13, wherein the activity of the bacteria or algae is stimulated by provision of an energy source, preferably the addition of glucose and/or fructose.

16. A sensor as claimed in any one of claims 10 to 15, wherein the bacteria or algae are retained on a translucent alumina filter.

17. A sensor as claimed in any of claims 10 to 16, which also comprises a control electrode in a sensor having aqueous liquid of predetermined purity, to allow the determination of normality by comparison with the electrical parameter output thereof with that of the electrode (4).

18. A sensor as claimed in any of claims 10 to 17, wherein electrical current is measured as derived by means of a carbon or platinum electrode poised in the region of 400-500 mV with reference to silver/silver chloride.

19. A sensor as claimed in any of claims 10 to 18, wherein the bacteria or algae are in a 0.2 µm bacteriological filter membrane held against the electrode surface by nylon mesh.

20. A sensor as claimed in any of claims 10 to 18, wherein the bacteria or algae are held in a reservoir through which the aqueous liquid and mediator can be passed prior to their passing to the electrode (4).

## Patentansprüche

1. Verfahren zum Nachweisen von Verschmutzung in einem wäßrigen Flüssigkeitsstrom, umfassend die Schritte: Zugeben eines verträglichen Elektronentransfervermittlers zu einem Teil der Flüssigkeit, Einleiten des Gemisches in eine Sensorzelle, in der lebende Bakterien oder Algen enthalten sind, Stimulieren einer Aktivität der Bakterien oder Algen, und Messen der Höhe der Aktivität an einer Elektrode in der Zelle mittels Elektronentransfers an diese durch den Vermittler; **dadurch gekennzeichnet**, daß der Vermittler Kaliumferricyanid, Dimethylbenzochinon oder p-Benzochinon, entweder allein oder als Mischung ist, und dadurch daß die beobachtete Aktivität der Bakterien oder Algen die Atmung und/oder Photosynthese ist.

2. Verfahren nach Anspruch 1, **dadurch gekennzeichnet**, daß der Vermittler durch Elektronentransfer von dem Atmungs- oder Photosyntheseweg der Bakterien zu der Elektrode wirkt, wobei der davon fließende Strom als Maß der Bakterienaktivität wirkt.

3. Verfahren nach Anspruch 1 oder 2, worin die Bakterien Cyanobakterien, vorzugsweise Synechococcus sind.

4. Verfahren nach Anspruch 1 oder 2, worin die Bakterien Eubakterien, vorzugsweise E. coli sind.

5. Verfahren nach Anspruch 1 oder 2, worin die Algen eukaryontische Algen, vorzugsweise Chlorella sind und der Vermittler nicht Kaliumferricyanid allein ist.

6. Verfahren nach Anspruch 3 oder 5, worin das Mittel zum Stimulieren der Aktivität der Bakterien oder Algen eine Lichtquelle ist, vorzugsweise eine helle Leuchtdiode, die in der Nähe der Bakterien oder Algen angebracht ist.

7. Verfahren nach Anspruch 4 oder 5, worin die Bakterien- oder Algenaktivität durch Bereitstellung einer Energiequelle, vorzugsweise die Zugabe von Glukose und/oder Fructose, stimuliert wird.

8. Verfahren nach einem der vorhergehenden Ansprüche, worin die Bakterien oder Algen auf einem durchscheinenden Aluminiumoxidfilter zurückgehalten werden.

9. Verfahren nach einem der vorhergehenden Ansprüche, worin eine Bestimmung des Normalzustandes durch Vergleich der Ausgabe eines elektrischen Parameters der Elektrode mit der einer Kontrollelektrode in einem Sensor mit einer wäßrigen Flüssigkeit von vorbestimmter Reinheit vorgenommen wird.

10. Sensor zum Nachweisen der Anwesenheit einer Verunreinigung in einer wäßrigen Flüssigkeit, welcher eine Elektrode (4), lebende Bakterien oder Algen, Mittel zum Stimulieren der Aktivität der Bakterien oder Algen (6), einen verträglichen Vermittler zum Übertragen einer Anzeige der Höhe der Aktivität der Bakterien oder Algen auf die Elektrode und Mittel zum Messen eines elektrischen Parameters der Elektrode, zur Bestimmung bezüglich des Normalzustandes der Bakterien/Algenaktivität umfaßt; **dadurch gekennzeichnet**, daß der Vermittler Kaliumferricyanid, Dimethylbenzochinon oder p-Benzochinon, entweder allein oder in Mischung, ist.

11. Sensor nach Anspruch 10, worin die Bakterien Cyanobakterien, vorzugsweise Synechococcus sind.

12. Sensor nach Anspruch 10, worin die Bakterien Eubakterien, vorzugsweise E. coli sind.

13. Sensor nach Anspruch 10, worin die Algen eukaryontische Algen, vorzugsweise Chlorella sind, und daß der Vermittler nicht Kaliumferricyanid allein ist.

14. Sensor nach Anspruch 11 oder 13, worin das Mittel zum Stimulieren der Aktivität der Bakterien oder Algen eine Lichtquelle ist, vorzugsweise eine helle Leuchtdiode (6), die in der Nähe der Bakterien oder Algen angebracht ist.

15. Sensor nach Anspruch 12 oder 13, worin die Aktivität der Bakterien oder Algen durch Bereitstellung einer Energiequelle, vorzugsweise durch die Zugabe von Glukose und/oder Fructose stimuliert wird.

16. Sensor nach einem der Ansprüche 10 bis 15, worin die Bakterien oder Algen auf einem durchscheinenden Aluminiumoxidfilter zurückgehalten werden.

17. Sensor nach einem der Ansprüche 10 bis 16, welcher auch eine Kontrollelektrode in einem Sensor mit einer wäßrigen Flüssigkeit von vorbestimmter Reinheit umfaßt, um die Bestimmung des Normalzustands durch Vergleich mit der Ausgabe eines elektrischen Parameters davon mit dem der Elektrode (4) zu ermöglichen.

18. Sensor nach einem der Ansprüche 10 bis 17, worin ein elektrischer Strom gemessen wird, der mittels einer Kohlenstoff- oder Platinelektrode abgeleitet ist, die sich im Bereich von 400 bis 500 mV, bezogen auf Silber/Silberchlorid, im Gleichgewicht befindet.

19. Sensor nach einem der Ansprüche 10 bis 18, worin die Bakterien oder Algen in einer 0,2 µm bakteriologischen Filtermembran sind, die gegen die Elektrodenoberfläche von einem Nylonnetz gehalten wird.

20. Sensor nach einem der Ansprüche 10 bis 18, worin die Bakterien oder Algen in einem Reservoir gehalten werden, durch welches die wäßrige Flüssigkeit und der Vermittler geleitet werden können, bevor sie zu der Elektrode (4) geleitet werden.

## Revendications

1. Méthode pour détecter la pollution dans un courant de liquide aqueux comprenant les étapes de : addition à une portion du liquide d'un médiateur du transfert d'électrons compatible, passage du mélange dans une cellule de détection qui renferme une algue ou une bactérie vivante, stimulation de l'activité de ladite bactérie ou algue, et mesure du niveau de ladite activité à une électrode dans la cellule au moyen du transfert d'électron par ledit médiateur ; caractérisée en ce que ledit médiateur est du ferricyanure de potassium, de la diméthylbenzoquinone ou de la p-benzoquinone, seuls ou en mélange, et en ce que l'activité détectée au niveau de la bactérie ou de l'algue est une respiration et/ou une photosynthèse.

2. Méthode comme revendiquée dans la revendication 1, caractérisée en ce que le médiateur opère par un transfert d'électrons depuis la voie respiratoire ou de photosynthèse de la bactérie vers l'électrode, le flux de courant partant de l'électrode constituant la mesure de l'activité de la bactérie.

3. Méthode comme revendiquée dans la revendication 1 ou 2, dans laquelle les bactéries sont des cyanobactéries, de préférence Synechococcus.

4. Méthode comme revendiquée dans la revendication 1 ou 2, dans laquelle les bactéries sont des Eubactéries, de préférence E. coli.

5. Méthode comme revendiquée dans la revendication 1 ou 2, dans laquelle les algues sont des algues eucaryotes, de préférence chlorella et le médiateur n'est pas du ferricyanure de potassium seul.

6. Méthode comme revendiquée dans la revendication 3 ou 5, dans laquelle le moyen pour stimuler l'activité des bactéries ou des algues est une source lumineuse, de préférence une diode émettant une lumière brillante disposée à proximité des bactéries ou des algues.

7. Méthode comme revendiquée dans la revendication 4 ou 5, dans laquelle l'activité des bactéries ou des algues est stimulée par fourniture d'une source d'énergie, de préférence addition de glucose et/ou fructose.

8. Méthode comme revendiquée dans l'une quelconque des revendications précédentes, dans laquelle les bactéries ou les algues sont retenues sur un filtre d'alumine transparent.

9. Méthode comme revendiquée dans l'une quelconque des revendications précédentes, dans laquelle on détermine la normalité en comparant le paramètre électrique sortant de l'électrode à celui d'une électrode témoin dans un détecteur ayant un liquide aqueux de pureté prédéterminée.

10. Détecteur pour détecter la présence d'un polluant dans un liquide aqueux, ledit détecteur comprenant une électrode (4), des algues ou bactéries vivantes, un moyen de stimulation de l'activité des bactéries ou algues (6), un médiateur compatible pour transmettre une indication du niveau d'activité des bactéries ou algues à ladite électrode et un moyen de mesure d'un paramètre électrique de ladite électrode pour déterminer la normalité de l'activité des bactéries/algues ; caractérisée en ce que ledit médiateur est du ferricyanure de potassium, de la diméthylbenzoquinone ou de la p-benzoquinone, seuls ou en mélange.

11. Détecteur comme revendiqué dans la revendication 10, dans lequel les bactéries sont des cyanobactéries, de préférence Synechococcus.

12. Détecteur comme revendiqué dans la revendication 10, dans lequel les bactéries sont des Eubactéries, de préférence E. coli.

13. Détecteur comme revendiqué dans la revendication 10, dans lequel les algues sont des algues eucaryotes, de préférence chlorella et le médiateur n'est pas du ferricyanure de potassium seul.

14. Détecteur comme revendiqué dans la revendication 11 ou 13, dans lequel le moyen pour stimuler l'activité des bactéries ou des algues est une source lumineuse, de préférence une diode émettant une lumière brillante (6) disposée à proximité des bactéries ou des algues.

15. Détecteur comme revendiqué dans la revendication 12 ou 13, dans lequel l'activité des bactéries ou des algues est stimulée par fourniture d'une source d'énergie, de préférence addition de glucose et/ou fructose.

16. Détecteur comme revendiqué dans l'une quelconque des revendications 10 à 15, dans lequel les bactéries ou les algues sont retenues sur un filtre d'alumine transparent.

17. Détecteur comme revendiqué dans l'une quelconque des revendications 10 à 16 qui comprend également une électrode témoin dans un détecteur ayant un liquide aqueux de pureté prédéterminée, pour permettre la détermination de la normalité par comparaison du paramètre électrique sortant de l'électrode témoin à celui de l'électrode (4).

18. Détecteur comme revendiqué dans l'une quelconque des revendications 10 à 17, dans lequel le courant électrique est mesuré comme dérivant d'une électrode de carbone ou de platine en équilibre dans la région de 400 - 500 mV par rapport à argent/chlorure d'argent.

19. Détecteur comme revendiqué dans l'une quelconque des revendications 10 à 18, dans lequel les bactéries ou algues sont dans une membrane biologique filtrante de 0,2 µm maintenue contre la surface de l'électrode par un filet de Nylon.

20. Détecteur comme revendiqué dans l'une quelconque des revendications 10 à 18, dans lequel les bactéries ou algues sont maintenues dans un réservoir au travers duquel le liquide aqueux et le médiateur peuvent passer avant de passer par l'électrode (4).
